(19) Europäisches Patentamt / European Patent Office / Office européen des brevets

(11) **EP 4 679 063 A1**

(12) **EUROPEAN PATENT APPLICATION**
published in accordance with Art. 153(4) EPC

(43) Date of publication:
14.01.2026 Bulletin 2026/03

(21) Application number: 25749107.6

(22) Date of filing: 20.01.2025

(51) International Patent Classification (IPC):
*G01N 21/45* (2006.01)   *G01N 21/31* (2006.01)
*G01N 21/47* (2006.01)   *G01B 9/02091* (2022.01)
*G01J 9/02* (2006.01)    *A61B 5/00* (2006.01)

(52) Cooperative Patent Classification (CPC):
A61B 5/00; G01B 9/02091; G01J 9/02;
G01N 21/31; G01N 21/45; G01N 21/47

(86) International application number:
PCT/KR2025/001080

(87) International publication number:
WO 2025/165020 (07.08.2025 Gazette 2025/32)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 31.01.2024  KR 20240015151
08.11.2024  KR 20240157639

(71) Applicant: **Korea Advanced Institute of Science and Technology**
**Daejeon 34141 (KR)**

(72) Inventors:
• **YOO, Hongki**
  **Daejeon 34141 (KR)**
• **LEE, Seung Eon**
  **Daejeon 34141 (KR)**

(74) Representative: **Manitz Finsterwald**
**Patent- und Rechtsanwaltspartnerschaft mbB**
**Martin-Greif-Straße 1**
**80336 München (DE)**

(54) **OPTICAL COHERENCE TOMOGRAPHY SYSTEM USING CHROMATIC ABERRATION, AND OPERATION METHOD THEREFOR**

(57)   An optical coherence tomography (OCT) system includes a light source assembly that generates original light, a sample arm that provides measurement light to a sample and provides measurement light reflected from the sample to an optical coupler, a reference arm of an optical structure that reflects a reference light, the optical coupler divides the original light into the measurement light and the reference light and provides them to the sample arm and the reference arm, respectively, and combines the reflected measurement light and the reflected reference light provided from the sample arm and the reference arm, respectively, to output combined light including an interference signal between the measurement light and the reference light, and an optical detector that analyzes the interference signal of the combined light provided from the optical coupler to generate an optical coherence tomographic image, wherein the sample arm includes a dispersive lens pair that induces chromatic focal shift of the measurement light by allowing the measurement light to have multiple different divergence angles depending on the wavelength.

FIG. 1

**Description**

**[Background Art]**

Optical coherence tomography (OCT) is an imaging technique that uses the interference phenomenon of light to obtain three-dimensional cross-sectional images of biological tissue. There is active development of high-resolution OCT with resolutions of 1-3 $\mu$m for the observation of fine tissue structures.

The high-resolution OCT with a resolution of 1-3 $\mu$m offers significant potential for subcellular diagnostics compared to conventional OCT with a resolution of 10-20 $\mu$m. However, the use of high numerical aperture (NA) lenses required for such high resolution inevitably reduces a depth of focus (DOF), limiting the practical application of the OCT. That is, when increasing the lateral resolution by using an objective lens with high NA, a problem occurs in which the DOF is limited in inverse proportion to the lateral resolution.

Several hardware- and software-based techniques have been proposed to resolve the tradeoff between lateral resolution and the DOF.

A representative hardware-based technique is to increase the depth of focus by forming a Bessel beam. For this purpose, special optical systems such as axicon lenses are used, resulting in the formation of the Bessel beam in which the focus of all light is lengthened regardless of wavelength, ultimately expanding the depth of focus.

However, when using Bessel beam optical systems, the system signal-to-noise ratio (SNR) decreases inversely with the square of the increased depth of focus, and this reduced system SNR significantly limits imaging deep into tissues.

Further, artifacts caused by the side lobes of the Bessel beam degrade the performance of the image.

On the other hand, software-based techniques such as interferometric synthetic aperture microscopy (ISAM) require a very high level of phase stability over a wide range of data, which limits their application in dynamic imaging.

**[Disclosure]**

**[Technical Problem]**

The present disclosure provides an optical coherence tomography (OCT) system and an operating method thereof that use a wide wavelength band of original light, intentionally induce a chromatic focal shift in a sample arm so that measurement light of different wavelengths is focused at different points axially on the sample to expand a depth of focus (DOF), and utilize image reconstruction algorithms appropriate to such systems to resolve the trade-off between lateral resolution and depth of focus while maintaining a high system signal-to-noise ratio (SNR).

**[Technical Solution]**

According to an aspect, the optical coherence tomography (OCT) system includes a light source assembly that generates original light, a sample arm that provides measurement light to a sample and provides measurement light reflected from the sample to an optical coupler, a reference arm of an optical structure that reflects a reference light, the optical coupler that divides the original light into the measurement light and the reference light and provides them to the sample arm and the reference arm, respectively, and combines the reflected measurement light and the reflected reference light provided from the sample arm and the reference arm, respectively, to output combined light including an interference signal between the measurement light and the reference light, and an optical detector that analyzes the interference signal of the combined light provided from the optical coupler to generate an optical coherence tomographic image, wherein the sample arm includes a dispersive lens pair that induces chromatic focal shift of the measurement light by allowing the measurement light to have multiple different divergence angles depending on the wavelength.

The optical detector may generate an optical coherence tomographic image based on a valid interference signal for each depth of the sample by applying a Gaussian window having a different center position depending on the depth of the sample.

The center position of the Gaussian window may be determined by the chromatic focal shift.

The chromatic focal shift may be expressed as the relationship between the wavelength and the focal shift formed on the sample, and in the Gaussian window, the wavelength of the point at which the focus is formed may be determined as the center position so that the interference signal having the wavelength of the point at which the focus is formed is used as a valid interference signal.

The optical detector may generate the optical coherence tomographic image by performing an operation using the interference signal obtained from the optical coupler, a complex function defined to have various frequencies depending on the depth of the sample in the k-domain and the Gaussian window.

The reference arm may include a dispersion compensator that compensates for the dispersion difference between the measurement light and the reference light caused by the dispersive lens pair.

The reference arm may include a polarization controller disposed at the forefront on the path of the reference light and adjusting the polarization between the sample arm and the reference arm, a collimator disposed behind the polarization controller and in front of the dispersion compensator to convert the reference light into parallel light, and a mirror disposed behind the dispersion compensator and reflecting the reference light.

The sample arm may further include the collimator disposed at the forefront on the path of the measurement light and in front of the dispersive lens pair, and converting the measurement light provided from the optical coupler into parallel light, a scanning mirror that reflects the measurement light dispersed from the dispersive lens pair in various directions and provides the measurement light to the sample, and provides the measurement light reflected from the sample to the dispersive lens pair through scanning, and an objective lens that focuses the measurement light onto the sample.

The light source assembly may include a light emitter that generates original light in the form of a supercontinuum laser with a broadband wavelength, a long pass filter (LPF) and a short pass filter (SPF) for filtering a target wavelength band from the original light, and a collimator that collimates original light of the target wavelength band that has passed through the long pass filter and the short pass filter and provides the source light to the optical coupler.

According to another aspect, an operating method of an optical coherence tomography (OCT) system includes dividing original light into measurement light and reference light through an optical coupler and providing the measurement light and reference light to a sample arm and to a reference arm, respectively, causing the measurement light, in which chromatic focal shift is induced, to be incident on the sample to be measured by a dispersive lens pair that induces chromatic focal shift of the measurement light by having a plurality of different divergence angles according to the wavelength through the sample arm, combining the measurement light reflected from the sample through the optical coupler and the reflected reference light provided from the reference arm to generate combined light including an interference signal between the measurement light and the reference light, and generating an optical coherence tomographic image by analyzing the interference signal through an optical detector.

The generating of the optical coherence tomographic image may include setting a Gaussian window having different center positions depending on the depth of the sample, and generating the optical coherence tomographic image based on a valid interference signal for each depth of the sample by applying the Gaussian window.

The Gaussian window may have a center position of the Gaussian window determined by the chromatic focal shift defined as the relationship between the wavelength and the focal shift formed on the sample.

The generating of the optical coherence tomography image may generate the optical coherence tomography image based on an interference signal in which the dispersion difference between the measurement light and the reference light caused by the dispersive lens pair is compensated for through the dispersion compensator of the reference arm.

The generating of the optical coherence tomographic image may include multiplying the interference signal with a complex exponential function having different frequencies with the Gaussian window and calculating a sum to calculate a complex coefficient for a particular frequency of the interference signal, repeating the process for all frequency ranges to generate the entire frequency spectrum of the interference signal, and calculating a magnitude of the entire frequency spectrum to generate the optical coherence tomographic image.

Before the providing step, the method may further include generating the original light through a light emitter which is a supercontinuum laser with a broadband wavelength.

**[Advantageous Effects]**

According to the present disclosure, high resolution and high signal-to-noise ratio (SNR) can be achieved at the expanded DOF through the OCT system and algorithm using chromatic focal shift, thereby obtaining high-resolution/high-sensitivity tomographic images.

**[Description of the Drawings]**

FIG. 1 illustrates a configuration diagram of an optical coherence tomography (OCT) system according to an embodiment.

FIG. 2 shows a Fourier transform algorithm for generating a conventional OCT image.

FIG. 3 shows the position of the focus according to the wavelength generated in a sample arm according to an embodiment of the present disclosure.

FIG. 4 illustrates a chromatic gating algorithm (ChG algorithm) for generating the OCT image according to an embodiment of the present disclosure.

FIG. 5 shows an imaging result of applying a Gaussian window to the interference signal (fringe) in FIG. 4.

FIG. 6 is a flowchart illustrating the operation of the OCT system according to an embodiment.

FIGS. 7A, 7B, 7C, and 7D show the results of imaging small beads in water using the OCT system that induced chromatic focal shift of the present disclosure and the ChG algorithm according to experiments.

FIGS. 8A, 8B, 8C, and 8D show the results of imaging small beads in water using conventional high-resolution OCT technology and a Fourier transform-based algorithm according to experiments.

FIG. 9 shows an imaging result obtained using the conventional high-resolution OCT system and the Fourier transform-based algorithm according to an experiment.

FIG. 10 is an imaging result obtained using the OCT system that induced chromatic focal shift of the present disclosure and ChG algorithm according to an experiment.

FIG. 11 is an imaging result obtained using the OCT system that induced chromatic focal shift of the present disclosure and the ChG algorithm according to an experiment.

FIG. 12 shows an imaging result obtained using the OCT system that induced chromatic focal shift of the present disclosure according to an experiment and the conventional Fourier transform-based algorithm.

FIG. 13 is a contrast comparison chart analyzed using the results of FIGS. 11 and 12.

**[Mode for Invention]**

The present disclosure will be described in detail hereinafter with reference to the accompanying drawings, in which embodiments of the present disclosure are shown. As those skilled in the art would realize, the described embodiments may be modified in various different ways, all without departing from the spirit or scope of the present disclosure. The drawings and description are to be regarded as illustrative in nature and not restrictive, and like reference numerals designate like elements throughout the specification.

In the description, unless explicitly stated to the contrary, the word "comprise" and variations such as "comprises" and "comprising" should be understood to imply the inclusion of stated elements but not the exclusion of any other elements.

Terms written in the present specification such as "unit," "module," and the like indicate a unit processing at least one or more functions or operations, and these functions or operations can be implemented by hardware or software, or a combination of hardware and software.

In the description, reference numerals and names are attached for convenience of explanation, and the devices are not necessarily strictly limited to the reference numerals or names.

In this specification, "transmitting or providing" may include not only direct transmission or provision, but also indirect transmission or provision via another device or by using a detour route.

In this specification, expressions described in the singular may be interpreted as singular or plural unless an explicit expression such as "one" or "single" is used.

In this specification, the same reference numerals refer to the same components regardless of the drawings, and "and/or" includes each and every combination of one or more of the above-mentioned components.

In this specification, while terms including ordinal numbers, such as "first" and "second," etc., may be used to describe various components, such components are not limited to the above terms. These terms are only used to distinguish one component from another component. For example, a first component may be referred to as a second component, and similarly, the second component may be referred to as the first component without departing from the range of the present disclosure.

In the flowchart described with reference to drawings in this description, the operation order may be changed, several operations may be merged, certain operations may be divided, and specific operations may not be performed.

The device of the present disclosure is a computing device configured and connected such that at least one processor can perform the operations of the present disclosure by executing instructions. A computer program includes instructions that cause a processor to perform the operations of the present disclosure and may be stored in a non-transitory computer-readable storage medium. The computer program may be downloaded over a network or sold in product form.

Hereinafter, in the description, the configuration of the related art is described without assigning reference numerals to distinguish it from the configuration of the present disclosure.

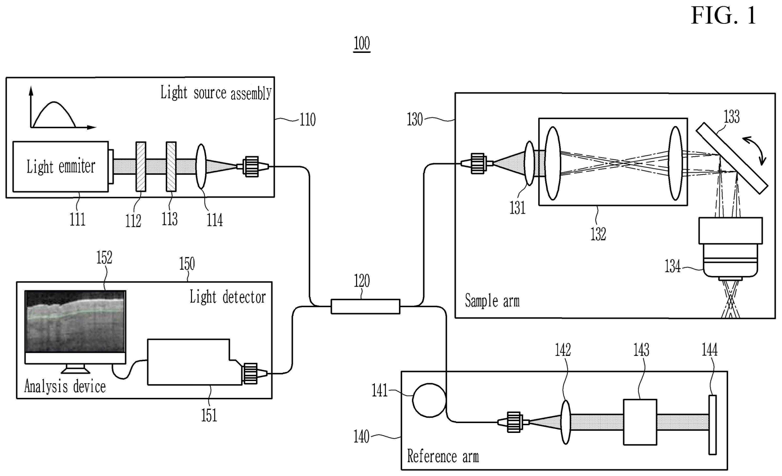

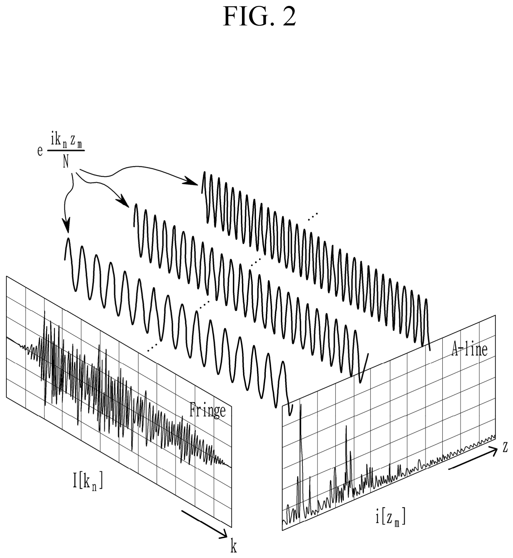

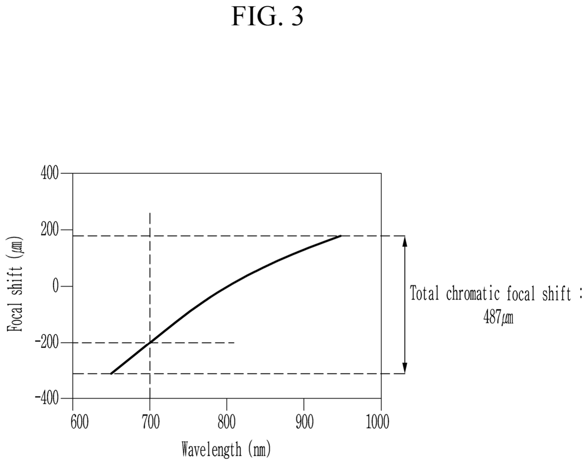

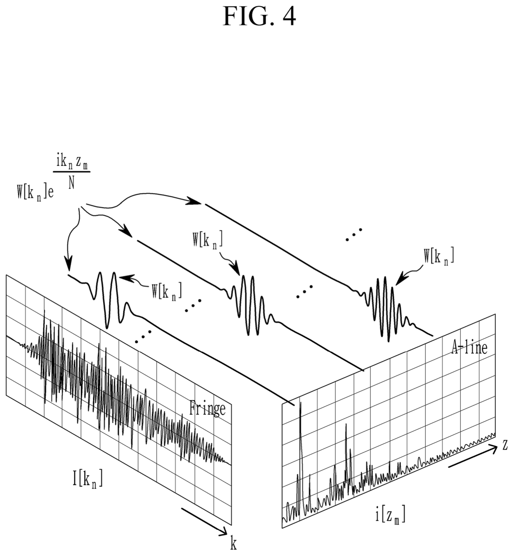

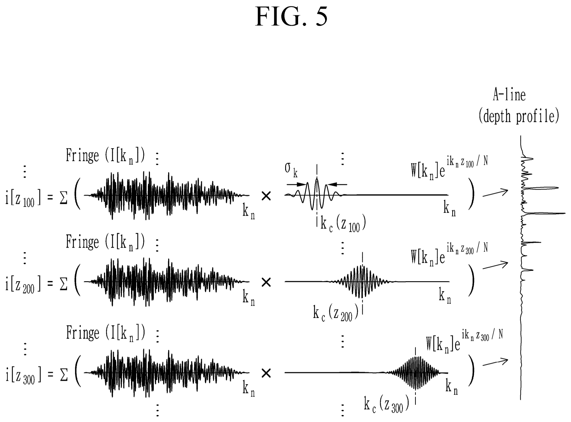

FIG. 1 illustrates a configuration diagram of an optical coherence tomography (OCT) system according to an embodiment, FIG. 2 shows a Fourier transform algorithm for generating a conventional OCT image, FIG. 3 shows the position of the focus according to the wavelength generated in a sample arm according to an embodiment of the present disclosure, FIG. 4 illustrates a chromatic gating algorithm (ChG algorithm) for generating the OCT image according to an embodiment of the present disclosure, and FIG. 5 shows an imaging result of applying a Gaussian window to the interference signal (fringe) in FIG. 4.

Referring to FIG. 1, an optical coherence tomography (OCT) system 100 includes a light source assembly 110, an interferometer 120, a sample arm 130, a reference arm 140, and an optical detector 150.

The light source assembly 110 is a component that generates light and may include a light emitter 111, a long pass filter (LPF) 112, a short pass filter (SPF) 113, and a collimator 114.

The light emitter 111 generates original light. The light emitter 111 is a original light and may generate a supercontinuum laser having a broadband wavelength.

The LPF 112 is an optical filter that filters light with a wavelength longer than a target wavelength band in order to filter the target wavelength band.

The SPF 113 is an optical filter that filters light with a wavelength shorter than the target wavelength band in order to filter the target wavelength band.

According to an embodiment, original light provided by the light emitter 111 may be filtered into original light in a spectral range between 650 nm and 950 nm by passing through the LPF 112 and the SPF 113.

The first collimator 114 is a lens that collects light of a broadband wavelength with high light collection efficiency. That is, the first collimator 114 collimates the original light to incident original light of the broadband wavelength that has passed through the LPF 112 and the SPF 113 into the interferometer 120.

The interferometer 120 is a component that separates light and generates interference light, and may include an optical fiber coupler 121.

The optical fiber coupler (hereinafter collectively referred to as an "optical coupler") 121 divides the original light incident from the first collimator 114 into measurement light and reference light, outputs the measurement light to the sample arm 130, and outputs the reference light to the reference arm 140. Additionally, the optical coupler 121 generates combined light by combining the measurement light and reference light reflected from the sample arm 130 and the reference arm 140, respectively, and outputs the combined light to the optical detector 150.

At this time, the measurement light and the reference light cause interference with each other within the combined light, and the combined light including the interference light is transmitted to the optical detector 150.

A 5:5 fiber coupler may be used as the optical coupler 121. That is, the original light may be divided at a division ratio of 50:50 ratio and provided to the sample arm 130 and the reference arm 140.

The sample arm 130 forms a path that provides the measurement light incident from the optical coupler 121 to the sample and outputs the measurement light reflected from the sample to the optical coupler 121. The sample arm 130 may include a second collimator 131, a dispersive lens pair 132, a scanning mirror 133, and an objective lens 134.

The second collimator 131 is a lens that is disposed on the path of the measurement light and converts the measurement light incident from the optical coupler 121 into parallel light.

The dispersive lens pair 132 includes two lenses having highly dispersive properties. The dispersive lens pair 132 is disposed on the path of the measurement light so that the measurement light incident from the second collimator 131 has a plurality of different divergence angles depending on the wavelength.

The scanning mirror 133 is disposed on the path of the measurement light, reflects the measurement light dispersed from the dispersive lens pair 132 and emits the measurement light to the sample, and changes the angle of the measurement light reflected from the sample and then emits the measurement light again through scanning.

At this time, the scanning mirror 133 may reflect the measurement light emitted from the dispersive lens pair 132 in various directions and emit the measurement light to the sample.

The objective lens 134 is a lens that focuses the measurement light on the sample. That is, the objective lens 134 serves to position a spot of measurement light on the sample.

Normally, in the absence of the dispersive lens pair 132, light of all wavelengths is focused to one point on the sample. In contrast, in the present disclosure, measurement light having various divergence angles-i.e., measurement light of various wavelengths-is focused at different points in the axial direction of the sample through the dispersive lens pair 132. Therefore, the measurement light is focused at different depths, which causes chromatic focal shift. This chromatic focal shift serves to extend the depth of focus (DOF), allowing high-resolution images of the sample to be obtained over a wide depth range.

The reference arm 140 forms a path for the reference light incident from the optical coupler 121. The reference arm 140 may include a polarization controller 141, a third collimator 142, a dispersion compensator 143, and a mirror 144.

The polarization controller 141 serves to adjust the polarization between the sample arm 130 and the reference arm 140.

The third collimator 142 is a lens that converts the reference light incident from the optical fiber coupler 121 into parallel light.

The dispersion compensator 143 compensates for the difference in dispersion between the measurement light and the reference light caused by the dispersive lens pair 132. To compensate for this dispersion difference, the dispersion compensator 143 is made of a circular glass plate made of the same material as the dispersive lens pair 132 and having a thickness equal to the sum of the thicknesses of the dispersive lens pair 132.

The mirror 144 reflects the reference light that has passed through the third collimator 142 and the dispersion compensator 143.

The optical detector 150 generates an optical coherence tomographic image from the combined light incident from the optical fiber coupler 121. The optical detector 150 may include a spectrometer 151 and an analysis device 152.

The spectrometer 151 detects an intensity signal according to the wavelength of the combined light incident from the optical fiber coupler 121 and outputs the detected signal to the analysis device 152.

The analysis device 152 interprets the detected signal received from the spectrometer 151 to generate an optical coherence tomographic image of the sample.

The optical coherence tomography operation by the above-described configuration is described as follows.

The original light of a broadband wavelength emitted from the light emitter 111 passes through the LPF 112 and the SPF 113 and is filtered into original light having a target wavelength band. The original light of the target wavelength band is collected through the first collimator 114 and provided to the optical coupler 121.

The optical coupler 121 divides the incident original light into measurement light and reference light. The interferometer 120 provides measurement light to the sample arm 130 and reference light to the reference arm 140.

The measurement light provided to the sample arm 130 is directed to the sample along a sample optical path of "second collimator 131 → dispersive lens pair 132 → scanning mirror 133 → objective lens 134."

The measurement light reflected from the sample is provided to the optical coupler 121 along the reverse path of the sample optical path-that is, the path of "objective lens 134 → scanning mirror 133 → dispersive lens pair 132 → second collimator 131."

The reference light provided to the reference arm 140 is provided to the mirror 144 along the reference optical path of "polarization controller 141 → third collimator 142 → dispersion compensator 143."

The reference light reflected from the mirror 144 is provided to the optical coupler 121 along the reverse path of the reference optical path-that is, the path of "dispersion compensator 143 → third collimator 142 → polarization controller 141."

The reflected measurement light and the reflected reference light are combined in the optical coupler 121 and provided to the spectrometer 151. At this time, the combined reflected measurement light and reflected reference light cause interference with each other. These interference signals are provided to the analysis device 152 in the form of intensity signals according to wavelength by the spectrometer 151.

The analysis device 152 performs an operation to convert the interference signal detected by the spectrometer 151 into a tomographic image, and this operation corresponds to the ChG algorithm described later. That is, the analysis device 152 interprets the interference signal in the form of an intensity signal according to the wavelength according to the ChG algorithm to generate a tomographic image.

According to an example, the original light of a supercontinuum laser passes through the LPF 112 and the SPF 113 and is incident on the optical coupler 121 as broadband light having a spectrum range between 650 nm and 950 nm. This original light is divided into measurement light and reference light at a division ratio of 50:50 by the optical coupler 121, and the measurement light is provided to the sample arm 130 and the reference light is provided to the reference arm 140.

In the sample arm 130, the measurement light may pass through the dispersive lens pair 132 including a pair of high-dispersion aspherical lenses, pass through the scanning mirror 133, and reach the objective lens 134 with a focal distance of 16.6 mm. This configuration may achieve a numerical aperture (NA) of 0.08 and chromatic focal shift of 487 $\mu$m with an input optical diameter of 2.7 mm.

A custom-designed dispersion compensator 143 is installed in the reference arm 140. The dispersion compensator 143 is made of a circular glass plate made of the same material as the dispersive lens pair 132 used in the sample arm 130. The dispersion compensator 143 may be twice as thick as the dispersive lens pair 132 to ensure matched dispersion characteristics between the sample arm 130 and the reference arm 140.

The interference signal reflected from the sample arm 130 and the reference arm 140 and provided to the spectrometer 151 through the optical coupler 121 may include a bandwidth of 300 nm, from 650 nm to 950 nm. The spectrometer 151 has 4096 pixels, providing a spectral resolution of 0.073 nm per pixel, which allows an axial imaging range of up to 2.11 mm in air.

The OCT system 100 uses a original light of a wide wavelength band and intentionally induces chromatic focal shift using the dispersive lens pair 132 in the sample arm 130 and the dispersion compensator 143. Therefore, the OCT system 100 may expand a depth of focus DOF by focusing measurement lights of different wavelengths at different points on the sample in the axial direction.

However, similar to when using conventional Bessel beams, system signal-to-noise ratio degradation and artifacts occur. Therefore, the analysis device 152 uses the ChG algorithm to further alleviate signal-to-noise ratio degradation and remove artifacts.

The ChG algorithm is a proposed algorithm of the present disclosure that utilizes changes that occur when chromatic focal shift is induced in the OCT system 100.

When using a typical optical system without chromatic focal shift, the interference signal formed in the optical detector has different frequencies in the wavenumber domain (k-domain) depending on the axial depth of the sample, but the envelope of the interference signal has no correlation with the depth of the sample.

However, when chromatic focal shift is present, the envelope of the interference signal also shifts in the wavenumber domain (k-domain) depending on the depth of the sample. That is, in the case of normal dispersion, the intensity of the interference signal generated by the sample close to the optical system is large in the long wavenumber (k) domain (or short wavelength region), and as the sample moves away in the axial direction, the intensity of the interference signal in the short wavenumber (k) domain (or long wavelength region) increases.

Fourier transform is a common method for extracting tomographic images from interference signals in optical coherence tomography. This method extracts frequency components included in the interference signal in all wavenumber (k) domains. Therefore, this method may be suitable for general optical coherence tomography systems that have constant intensity interference signals in the wavenumber (k) domain regardless of the depth of the sample.

However, when there is chromatic focal shift, interference signals are formed only in a specific part of the wavenumber (k) domain depending on the depth of the sample, and the general Fourier transform uses not only the valid signals of the interference signals but also the invalid signals when generating an optical coherence tomographic image, which causes unnecessary artifacts and noise.

Therefore, the ChG algorithm may remove unnecessary noise and artifacts by adding a Gaussian window to the part corresponding to the valid interference signal created by the sample of the depth corresponding to each frequency during the Fourier transform process, thereby utilizing only the valid signals of the interference signal and discarding invalid signals. This increases the signal-to-noise ratio (SNR) and increases the system sensitivity.

FIG. 2 shows the Fourier transform result of the interference signal.

The fringe data in FIG. 2 is an interference signal provided from a spectrometer, and represents the interference signal according to wavenumber (k). A-Line data representing the depth profile of the sample is derived from the fringe data through Fourier transform. This process may be expressed mathematically as Equation 1 below.

[Equation 1]

$$i[z_m] = \sum_{n=1}^{N} I[k_n] e^{\frac{ik_n z_m}{N}}$$

$i[z_m]$ represents the final result of the Fourier transform-i.e., the A-line. The A-line corresponds to the depth profile at one point. $z_m$ represents the depth of the sample.

$I[k_n]$ is a fringe, which represents the interference signal after dispersion compensation and k-linearization.

$e^{\frac{ik_n z_m}{N}}$ represents a complex function with different frequencies depending on $z_m$ in the wavenumber domain (k-domain).

In the absence of chromatic focal shift, interference signals are evenly distributed across the fringes, regardless of the depth of the sample.

On the other hand, when chromatic focal shift is induced, the interference signal from the sample at a certain depth is concentrated in a region of a certain wavelength (or a certain wavenumber (k)).

Referring to FIG. 3, the position of the focus according to the wavelength generated in the sample arm 130 is shown, the horizontal axis is the wavelength (nm), the vertical axis is the focal shift ($\mu$m), and the total induced chromatic focal shift is 487 $\mu$m.

For example, if the 700 nm measurement light is focused at the -200 $\mu$m point, the measurement light reflected at the -200 $\mu$m point of the sample has a large intensity near 700 nm. Therefore, when a general Fourier transform such as that in FIG. 2 is performed, invalid signals are used at a certain depth, which causes artifacts and noise. To avoid these problems, a Gaussian window ($W[k_n]$) is used.

To generate a signal at the -200 $\mu$m point in the A-line, it is appropriate to use an interference signal near 700 nm. For this purpose, when generating a signal at the -200 $\mu$m point, a Gaussian window ($W[k_n]$) centered at the 700 nm point is used.

Similarly, the measurement light reflected at the 0 $\mu$m point of the sample has a large intensity near 800 nm. Therefore, when generating a signal at the 0 $\mu$m point, a Gaussian window ($W[k_n]$) centered at the 800 nm point is used.

That is, the Gaussian window ($W[k_n]$) has different center positions depending on the sample depth.

Referring to FIG. 4, the analysis device 152 applies the ChG algorithm to the interference signal provided from the spectrometer 151-that is, the fringe-to derive the A-line. This process may be expressed mathematically as Equation 2 below.

[Equation 2]

$$i[z_m] = \sum_{n=1}^{N} I[k_n]W[k_n]e^{\frac{ik_n z_m}{N}}$$

$$W[k_n] = e^{-\frac{1}{2}\left(\frac{k_n - k_c}{\sigma_k}\right)^2}$$

$$k_c = \frac{2\pi}{\lambda(z_m)}$$

$i[z_m]$ represents the A-line, which is the final result of the ChG algorithm.

$I[k_n]$, $e^{\frac{ik_n z_m}{N}}$ is the same as defined in Equation 1.

$W[k_n]$ represents the Gaussian window.

$k_c$ represents the center position of the Gaussian window and is a function of $z_m$. The function $\lambda(z_m)$ represents the wavelength of the measurement light focused at the depth of $z_m$.

The analysis device 152 may obtain $\lambda(z_m)$ through various embodiments.

According to an embodiment, the analysis device 152 analyzes the beam profile of the measurement light reflected from the sample arm 130 through simulation of optical modeling software. For example, optical modeling software such as Optics Studio (Zemax) may be used. This optical modeling software may simulate the output optical characteristics of the sample arm and quantify the chromatic focal shift.

The analysis device 152 may obtain $\lambda(z_m)$ by checking at which position of the sample the measurement light of each wavelength is focused through optical modeling software.

According to another embodiment, the operator may obtain $\lambda(z_m)$ through a mirror imaging experiment, convert it into a database, and store it in the analysis device 152. The operator may obtain $\lambda(z_m)$ by placing a mirror at each $z_m$ position on the sample and determining which wavelength of measurement light is detected most strongly.

According to another embodiment, the operator may manually adjust the $k_c(z_m)$ while taking random samples to select the $k_c(z_m)$ that produces the sharpest results, which may then be converted into a database and stored in the analysis device 152.

$\sigma_k$ represents the width of the Gaussian window.

According to an example, the operator may manually adjust the $\sigma_k$ while taking random samples to select the $\sigma_k$ that produces the sharpest results, which may then be converted into a database and stored in the analysis device 152.

The process of deriving the A-line from the fringe by applying the Gaussian window ($W[k_n]$) described with reference to Equation 2 is illustrated in an example in FIG. 5.

Referring to FIG. 5, the center ($k_c$) of the Gaussian window ($W[k_n]$) is disposed at different positions depending on the frequency, and the process of deriving the A-line from the fringe by applying this Gaussian window ($W[k_n]$) is shown.

The position of the center ($k_c$) of the Gaussian window ($W[k_n]$) is determined by chromatic focal shift, as described in FIG. 3. Therefore, it is possible to generate an optical coherence tomographic image based on the valid interference signal actually reflected at that depth through the Gaussian window.

As described above, the OCT system 100 minimizes potential drawbacks while utilizing the advantages of axial chromatic focal shift. First, the OCT system 100 may expand the DOF while maintaining high lateral resolution by utilizing the optical system 131, 132, 133, and 134 of the sample arm 130 with chromatic focal shift and high NA. Second, high axial resolution is ensured by using a broadband light source 110 and the spectrometer 151. Third, it is possible to effectively reduce noise and mitigate artifacts to optimize overall system performance.

The dispersive lens pair 132 of the sample arm 130 is disposed in front of the scanning mirror 133, through which diverging and converging light are generated for longer and shorter wavelengths while the measurement light of the central wavelength remains in a parallel state. This measurement light passes through the scanning mirror 133 and a high NA objective lens 134 to create a narrow and elongated focus by focusing at various axial positions so that an effective NA of 0.08 and a chromatic focal shift of 487 $\mu$m may be set.

To match the dispersion characteristics between the sample arm 130 and the reference arm 140, the dispersion compensator 143 made of the same material and thickness as the dispersive lens pair 132 of the sample arm 130 is disposed in the reference arm 140.

Additionally, high axial resolution and wide chromatic focal shift are achieved by using a light source with a wide bandwidth, in the range of 650 to 950 nm.

The analysis device 152 uses the ChG algorithm that utilizes the co-relationship between depth and wavenumber (k) as described in FIG. 4.

In conventional OCT systems without chromatic focal shift, the frequency of the interference signal varies with sample depth, but its envelope is not correlated with depth.

On the contrary, chromatic focal shift causes the envelope to shift in the wavenumber domain (k-domain) with sample depth. Assuming that chromatic focal shift is due to normal dispersion, the signal of a sample closer to the objective lens 134 shows higher intensity in the higher wavenumber (k) (or shorter wavelength) domain, and this trend shifts to the lower wavenumber (k) (or longer wavelength) domain as the sample distance increases. Outside of this domain, there is only noise with no valid signal.

As described in FIG. 2, Fourier transform is commonly used for image reconstruction in OCT and extracts signals in all wavenumber (k) domains regardless of sample depth. This Fourier transform is suitable for standard OCT systems, but in OCT systems with chromatic focal shift, the Fourier transform uses both valid and invalid signal regions, resulting in unnecessary artifacts and noise.

As described in FIGS. 3 to 5, the ChG algorithm of the present disclosure may reduce excessive noise by adding the Gaussian window that is variably centered and moves according to the frequency of the harmonic wave, thereby using only the effective wavenumber (k) domain that is in focus for image extraction.

This wavenumber (k)-based filtering mechanism-that is, the ChG algorithm-may mitigate three types of noise. First, the ChG algorithm may effectively suppress system-specific noise while minimizing signal loss. Second, the ChG algorithm may mitigate artifacts caused by out-of-focus light, similar to side-lobe artifacts observed in Bessel beam OCT. Third, the ChG algorithm may remove multiple scattered light that interferes with imaging of deep tissues. Such noise mitigation may increase the SNR and improve the visualization of deeper tissue structures.

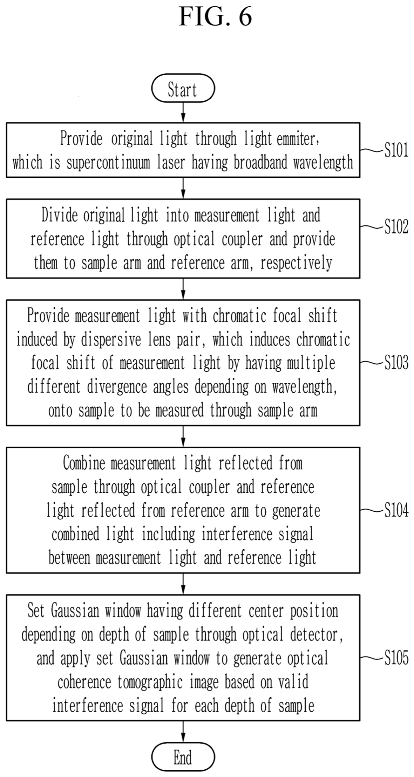

FIG. 6 is a flowchart illustrating the operation of the OCT system according to an embodiment, which describes the operation based on the components described in FIGS. 1 to 5.

Referring to FIG. 6, the OCT system 100 provides a original light through the light emitter 111, which is a supercontinuum laser having a broadband wavelength (S101).

The OCT system divides the original light into measurement light and reference light through the optical coupler 120 and provides them to the sample arm 130 and the reference arm 140, respectively (S102).

The OCT system provides measurement light with chromatic focal shift induced by the dispersive lens pair 132 onto the sample to be measured through the sample arm 130 (S103). Here, the dispersive lens pair 132 induces chromatic focal shift of the measurement light by having multiple different divergence angles depending on the wavelength.

The OCT system combines the measurement light reflected from the sample through the optical coupler 120 and the reference light reflected from the reference arm 140 to generate combined light including an interference signal between the measurement light and the reference light (S104).

The OCT system sets a Gaussian window having a different center position depending on the depth of the sample through the optical detector 150, and applies the set Gaussian window to generate an optical coherence tomographic image based on a valid interference signal for each depth of the sample (S105).

The Gaussian window may have a center position of the Gaussian window determined by the chromatic focal shift defined as the relationship between the wavelength and the focal shift formed on the sample.

In S105, the OCT system may generate the optical coherence tomographic image based on an interference signal in which the dispersion difference between the measurement light and the reference light caused by the dispersive lens pair is compensated for through the dispersion compensator of the reference arm.

At this time, the OCT system may perform a process of multiplying the interference signal with a complex exponential function having different frequencies with the Gaussian window and calculating a sum to calculate a complex coefficient for a particular frequency of the interference signal, repeat the process for all frequency ranges to generate the entire frequency spectrum of the interference signal, and calculate a magnitude of the entire frequency spectrum to generate the optical coherence tomographic image.

**Experimental Example**

According to the experiment, the imaging results obtained through the OCT system using the chromatic focal shift of the present disclosure and the ChG algorithm by photographing a 1 $\mu$m-sized bead were compared with the imaging results obtained through the conventional high-resolution OCT system, and the axial/lateral resolution and signal-to-noise ratio of the system were analyzed by analyzing the axial and lateral profiles and maximum intensity of the bead image. Further, the effectiveness of the OCT system using the chromatic focal shift of the present disclosure and the ChG algorithm were compared with a conventional high-resolution OCT system through tissue sample imaging, and the effectiveness of the ChG algorithm and the conventional Fourier transform algorithm in the OCT system using chromatic focal shift was compared.

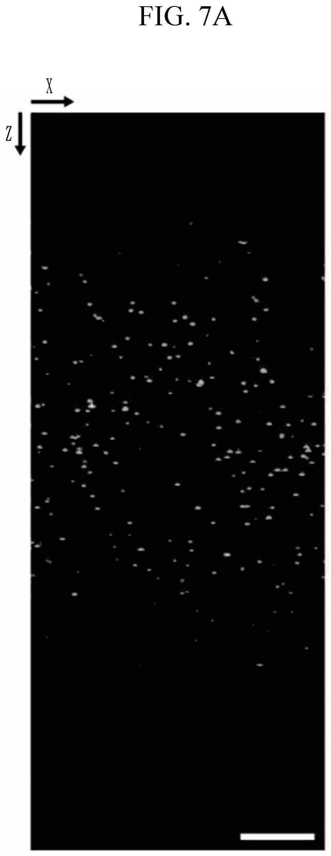

The OCT system 100 of the present disclosure may obtain an expanded DOF compared to conventional high-resolution OCT. These effects were confirmed through FIGS. 7A, 7B, 7C, 8A, 8B, 8C, 9, and 10.

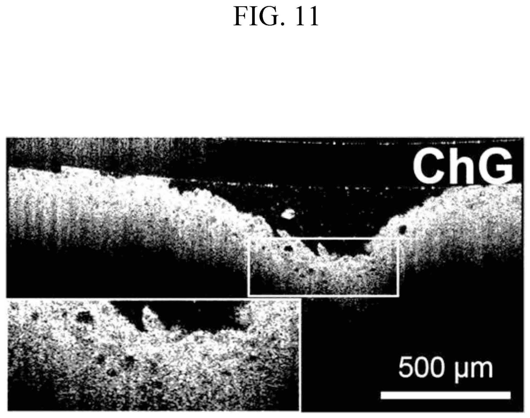

The ChG algorithm of the present disclosure has the effect of increasing SNR by removing noise inherent in the system and noise caused by multiple scattering, and removing side-lobe artifacts. These effects were confirmed through FIGS. 11, 12, and 13.

It was confirmed that the OCT system 100 of the present disclosure achieves isotropic high-resolution of 2-3 $\mu$m and shows up to 8 times improvement in depth of field DOF.

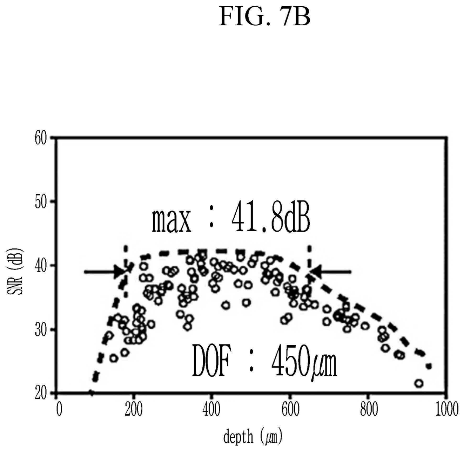

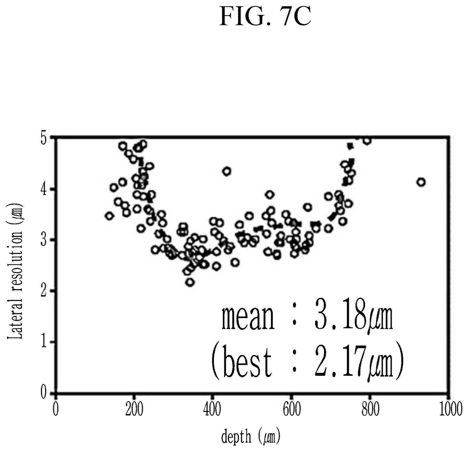

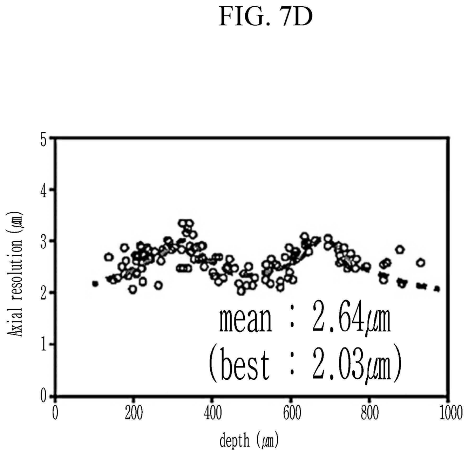

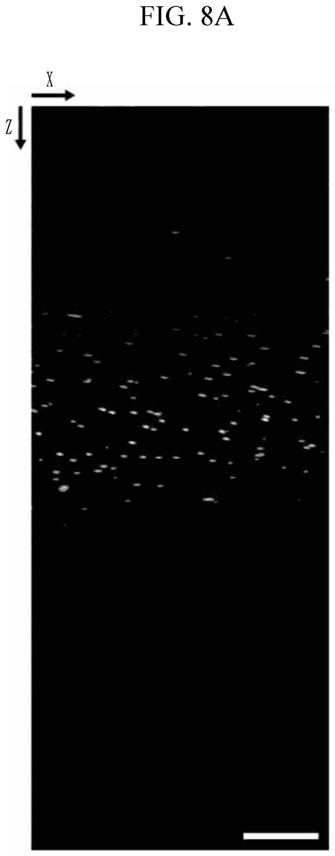

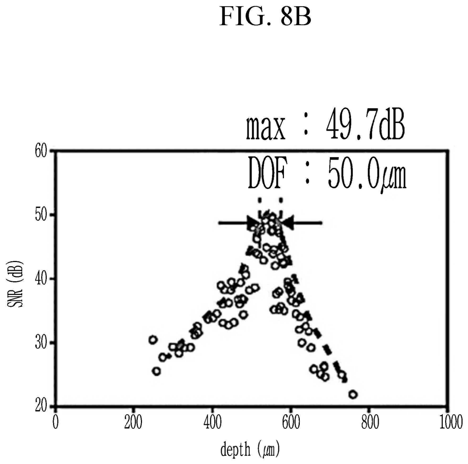

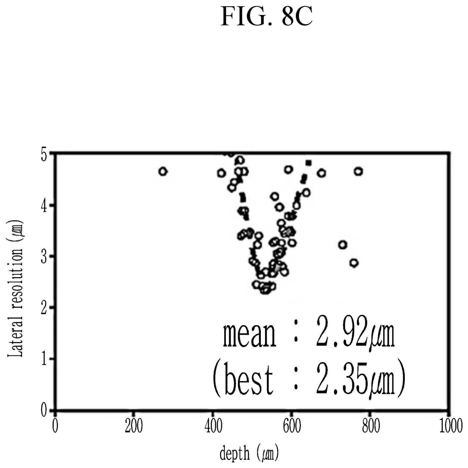

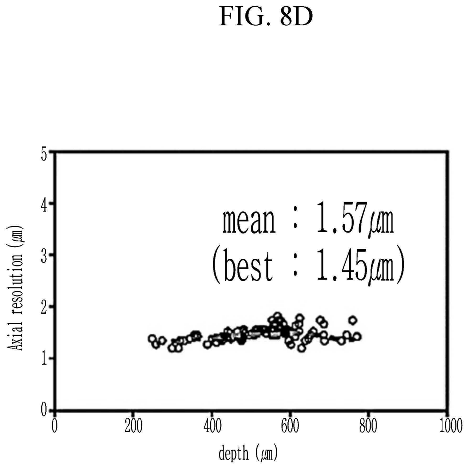

FIGS. 7A, 7B, 7C, and 7D show photographs of results of imaging small beads in water using the OCT system using chromatic focal shift of the present disclosure and the ChG algorithm, and FIGS. 8A, 8B, 8C, and 8D show photographs of the results of imaging small beads in water using the conventional Fourier transform-based high-resolution OCT technology.

To ensure an unbiased comparison, the experimental setup was designed to be easily switchable between the configuration of the OCT system 100 of the present disclosure and a conventional high-resolution OCT system. This switching design was achieved by selectively including or excluding the dispersive lens pair 132 and the dispersion compensator 143. This design allows for a direct and fair comparison of performance indicators between the two systems under identical experimental conditions.

FIGS. 7A, 7B, 7C, 7D and FIGS. 8A, 8B, 8C, 8D show photographs of imaging results of polystyrene microbeads with a diameter of about 0.99 $\mu$m suspended in water.

System performance was numerically analyzed based on SNR, lateral resolution, and axial resolution along the imaging depth.

FIGS. 7A and 8A show cross-sectional B-scan images of the beads.

FIGS. 7B and 8B show the relationship between depth ($\mu$m) and SNR (dB), FIGS. 7C and 8C show the relationship between depth ($\mu$m) and lateral resolution, and FIGS. 7D and 8D show the relationship between depth ($\mu$m) and axial resolution.

Consistent with the observation results of FIGS. 7A and 8A, according to FIGS. 7B and 8B, the OCT of the present disclosure showed the DOF of 450 $\mu$m, which is significantly expanded compared to the 50.0 $\mu$m result of the conventional OCT.

According to FIGS. 7C and 7D and FIGS. 8C and 8D, the OCT of the present disclosure maintained high lateral resolution throughout the enhanced DOF compared to conventional OCT, but the axial resolution was slightly deteriorated due to a reduced effective bandwidth of the interference signal.

Nevertheless, by using a light source with sufficient spectrum bandwidth, the reduced axial resolution may be maintained at a level similar to the lateral resolution, allowing the OCT of the present disclosure to achieve an isotropic resolution of 2-3 $\mu$m.

Additionally, the peak SNR of the OCT of the present disclosure is reduced compared to conventional high-resolution OCT, but maintains a high SNR over a significantly expanded depth range.

The results of the comparison of key performance indicators of the OCT of the present disclosure and the conventional OCT described above are summarized in Table 1. The performance of each system was evaluated at various imaging depths in terms of SNR, lateral resolution, and axial resolution based on numerical analysis, and Table 1 shows the results of the performance comparison.

(Table 1)

|  | High-resolution OCT | Chromatic OCT |
| --- | --- | --- |
| DOF [$\mu$m] | 50.0 | 450 |
| Peak SNR [dB] | 49.7 | 41.8 |
| Mean lateral resolution [$\mu$m] | 2.92 | 3.18 |
| Mean axial resolution [$\mu$m] | 1.57 | 2.64 |

According to the above experiments, it can be seen that the OCT system using chromatic focal shift of the present disclosure and ChG algorithm are more effective than the conventional Fourier transform-based high-resolution OCT. High-resolution imaging experiments using biological tissues further demonstrate the advantages of chromatic focal shift OCT, showing high SNR and minimal artifacts over an expanded imaging range.

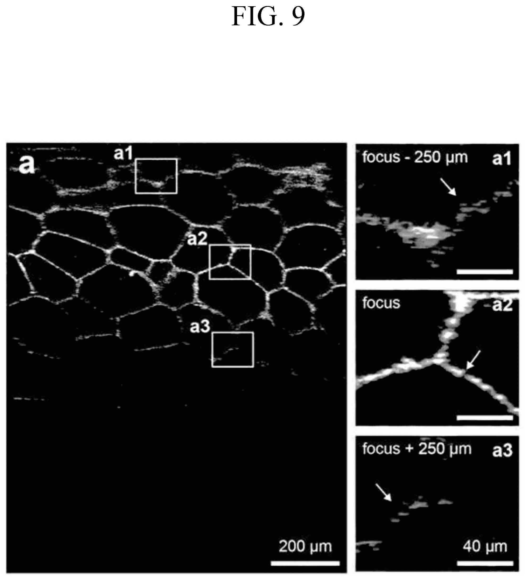

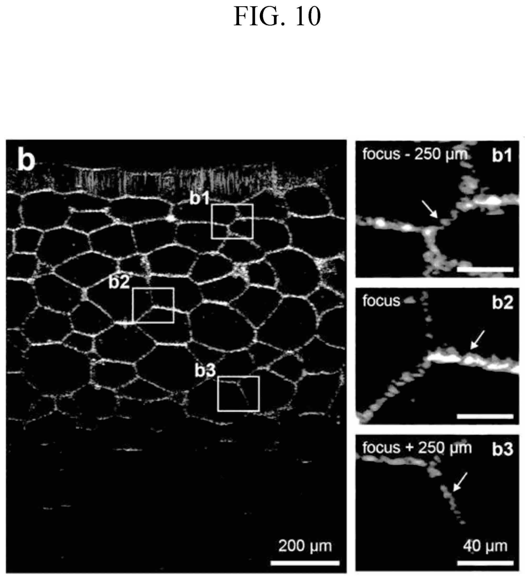

To demonstrate the practicality of the OCT system 100 of the present disclosure, an in vitro imaging experiment was performed on a fresh tissue sample-that is, a lemon sample-using the OCT system 100. At this time, the imaging results obtained by comparing the lemon sample with the conventional high-resolution OCT and the OCT of the present disclosure are shown in FIGS. 9 and 10.

FIG. 9 and FIG. 10 are cross-sectional images of a lemon sample according to an experiment of the present disclosure, wherein FIG. 9 is an imaging result obtained using the conventional high-resolution OCT, and FIG. 10 is an imaging result obtained using the OCT of the present disclosure.

Referring to a2 in FIG. 9, in the conventional high-resolution OCT image, the cell wall is clearly defined near the focal plane.

In contrast, referring to a1 and a3 of FIG. 9, in the conventional high-resolution OCT image, the cell wall appears blurry and wide in the region 250 μm away from the focus. These contents are highlighted by arrows. That is, it can seen that the cell wall is clear near the focus, but becomes blurry as it moves away from the focus.

In contrast, referring to b1, b2, and b3 in FIG. 10, it can be seen that the chromatic focal shift OCT images consistently display the cellular structure clearly overall, including regions far from the focal plane, as highlighted by arrows. That is, it can be seen that high SNR and resolution are maintained not only near the focus but also far from the focus.

Through these experiments, it can be seen that the OCT of the present disclosure may obtain images with high SNR and high resolution (lateral resolution and axial resolution) across a wider DOF.

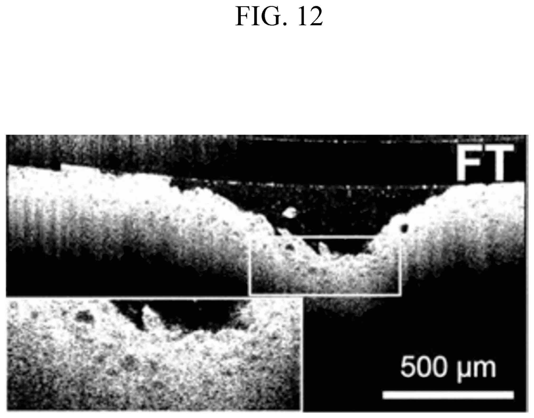

To demonstrate the practicality of the OCT system 100 of the present disclosure, a pig esophagus was photographed using the OCT system 100 of the present disclosure. The data captured in this manner were converted into images using the ChG algorithm of the present disclosure and the conventional Fourier transform algorithm, and are shown in FIGS. 11 and 12.

FIG. 11 and FIG. 12 show cross-sectional images of freshly excised porcine esophageal tissue taken using the OCT system using chromatic focal shift of the present disclosure, wherein FIG. 11 is an imaging result obtained using the ChG algorithm of the present disclosure, and FIG. 12 is an imaging result obtained using the conventional Fourier transform algorithm.

Here, ChG refers to the ChG algorithm of the present disclosure, and FT refers to the conventional Fourier transform algorithm.

According to FIG. 11, glycogen-rich squamous epithelial cells may be clearly identified. On the other hand, compared to FIG. 11, FIG. 12 shows more noise and lower clarity of cell structure.

That is, it was confirmed that FIG. 11 may image squamous epithelial cells of the tissue more clearly than FIG. 12. In particular, FIG. 11 shows significantly improved image quality in visualizing mature squamous epithelial cells with glycogen-rich cytoplasm. It can be seen that these enhancements effectively suppress multiple scattered light and noise within highly scattering media.

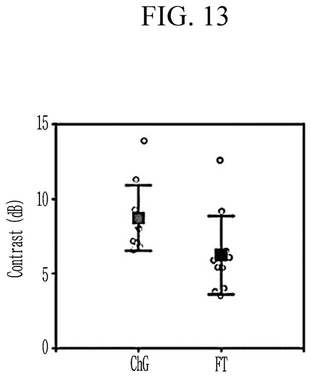

FIG. 13 is the result of analyzing the contrast through the difference in internal and external signal intensity of 10 squamous epithelial cells of FIGS. 11 and 12.

According to FIG. 13, the algorithm of the present disclosure has an average contrast of 8.72 dB (standard deviation 2.19 dB), while the conventional FT algorithm has an average contrast of 6.24 dB (standard deviation 2.62 dB). That is, it can be seen that the algorithm of the present disclosure increases the average contrast by 2.48 dB (39.7%) compared to the conventional FT algorithm. This is particularly significant considering the contrast of deep tissue images, which is typically limited to a few decibels.

These results show that the ChG algorithm of the present disclosure effectively removes noise caused by multiple scattering.

The embodiments of the present disclosure described above are not implemented only through devices and methods, but may also be implemented through a program that realizes a function corresponding to the configuration of the embodiments of the present disclosure or a recording medium on which the program is recorded.

While this disclosure has been described in connection with what is presently considered to be practical embodiments, it should be understood that the disclosure is not limited to the disclosed embodiments, but, on the contrary, is intended to cover various modifications and equivalent arrangements included within the spirit and scope of the appended claims.

**Claims**

1. An optical coherence tomography (OCT) system, comprising:

   a light source assembly that generates original light;
   a sample arm that provides measurement light to a sample and provides measurement light reflected from the sample to an optical coupler;
   a reference arm of an optical structure that reflects a reference light;
   the optical coupler that divides the original light into the measurement light and the reference light and provides them to the sample arm and the reference arm, respectively, and combines the reflected measurement light and the reflected reference light provided from the sample arm and the reference arm, respectively, to output combined light including an interference signal between the measurement light and the reference light; and an optical detector that analyzes the interference signal of the combined light provided from the optical coupler to generate an optical coherence tomographic image,

wherein the sample arm comprises a dispersive lens pair that induces chromatic focal shift of the measurement light by allowing the measurement light to have multiple different divergence angles depending on the wavelength.

2. The optical coherence tomography (OCT) system of claim 1, wherein the optical detector generates an optical coherence tomographic image based on a valid interference signal for each depth of the sample by applying a Gaussian window having a different center position depending on the depth of the sample.

3. The optical coherence tomography (OCT) system of claim 2, wherein the center position of the Gaussian window is determined from the chromatic focal shift.

4. The optical coherence tomography (OCT) system of claim 3, wherein the chromatic focal shift is expressed as the relationship between the wavelength and the focal shift formed on the sample, and in the Gaussian window, the wavelength of the point at which the focus is formed is determined as the center position so that the interference signal having the wavelength of the point at which the focus is formed is used as a valid interference signal.

5. The optical coherence tomography (OCT) system of claim 4, wherein the optical detector generates the optical coherence tomographic image by performing an operation using the interference signal obtained from the optical coupler, a complex function defined to have various frequencies depending on the depth of the sample in the k-domain, and the Gaussian window.

6. The optical coherence tomography (OCT) system of claim 1, wherein the reference arm comprises a dispersion compensator that compensates for the dispersion difference between the measurement light and the reference light caused by the dispersive lens pair.

7. The optical coherence tomography (OCT) system of claim 6, wherein the reference arm further comprises a polarization controller disposed at the forefront on the path of the reference light and adjusting the polarization between the sample arm and the reference arm;

a collimator disposed behind the polarization controller and in front of the dispersion compensator to convert the reference light into parallel light; and
a mirror disposed behind the dispersion compensator and reflecting the reference light.

8. The optical coherence tomography (OCT) system of claim 6, wherein the sample arm further comprises a collimator disposed at the forefront on the path of the measurement light and in front of the dispersive lens pair, and converting the measurement light provided from the optical coupler into parallel light;

a scanning mirror that reflects the measurement light dispersed from the dispersive lens pair in various directions and provides the measurement light to the sample, and provides the measurement light reflected from the sample to the dispersive lens pair through scanning; and
an objective lens that focuses the measurement light onto the sample.

9. The optical coherence tomography (OCT) system of claim 1, wherein the light source assembly comprises a light source that generates original light in the form of a supercontinuum laser with a broadband wavelength;

a long pass filter (LPF) and a short pass filter (SPF) for filtering a target wavelength band from the original light; and
a collimator that collimates original light of the target wavelength band that has passed through the long pass filter and the short pass filter and provides the original light to the optical coupler.

10. An operating method of an optical coherence tomography (OCT) system, comprising:

dividing original light into measurement light and reference light through an optical coupler and providing the measurement light and reference light to a sample arm and to a reference arm, respectively;
causing the measurement light, in which chromatic focal shift is induced, to be incident on the sample to be measured by a dispersive lens pair that induces chromatic focal shift of the measurement light by having a plurality of different divergence angles according to the wavelength through the sample arm;

combining the measurement light reflected from the sample through the optical coupler and the reflected reference light provided from the reference arm to generate combined light including an interference signal between the measurement light and the reference light; and

generating an optical coherence tomographic image by analyzing the interference signal through an optical detector.

11. The method of claim 10, wherein the generating of the optical coherence tomographic image comprises setting a Gaussian window having different center positions depending on the depth of the sample; and generating the optical coherence tomographic image based on a valid interference signal for each depth of the sample by applying the Gaussian window.

12. The method of claim 11, wherein the Gaussian window has a center position of the Gaussian window determined by the chromatic focal shift defined as the relationship between the wavelength and the focal shift formed on the sample.

13. The method of claim 12, wherein the generating of the optical coherence tomographic image generates the optical coherence tomographic image based on an interference signal in which the dispersion difference between the measurement light and the reference light caused by the dispersive lens pair is compensated for through a dispersion compensator of the reference arm.

14. The method of claim 13, wherein the generating of the optical coherence tomographic image comprises multiplying the interference signal with a complex exponential function having different frequencies with the Gaussian window and calculating a sum to calculate a complex coefficient for a particular frequency of the interference signal; repeating the process for all frequency ranges to generate the entire frequency spectrum of the interference signal; and calculating a magnitude of the entire frequency spectrum to generate the optical coherence tomographic image.

15. The method of claim 10, wherein before the providing step, the method further comprises generating the original light through a light source which is a supercontinuum laser with a broadband wavelength.

FIG. 1

100

Light source assembly 110

Light emmiter

111 112 113 114

Light detector 150
152

Analysis device

151

120

130 Sample arm

133

131
132
134

140 Reference arm

141 142 143 144

EP 4 679 063 A1

14

# FIG. 2

# FIG. 3

# FIG. 4

# FIG. 5

# FIG. 6

```
                        ( Start )
                            │
                            ▼
┌──────────────────────────────────────────────┐
│   Provide original light through light emmiter,│ ── S101
│ which is supercontinuum laser having broadband wavelength│
└──────────────────────────────────────────────┘
                            │
                            ▼
┌──────────────────────────────────────────────┐
│   Divide original light into measurement light and│ ── S102
│  reference light through optical coupler and provide│
│   them to sample arm and reference arm, respectively│
└──────────────────────────────────────────────┘
                            │
                            ▼
┌──────────────────────────────────────────────┐
│  Provide measurement light with chromatic focal shift│
│  induced by dispersive lens pair, which induces chromatic│ ── S103
│    focal shift of measurement light by having multiple│
│   different divergence angles depending on wavelength,│
│    onto sample to be measured through sample arm│
└──────────────────────────────────────────────┘
                            │
                            ▼
┌──────────────────────────────────────────────┐
│       Combine measurement light reflected from│
│     sample through optical coupler and reference│ ── S104
│     light reflected from reference arm to generate│
│         combined light including interference signal│
│       between measurement light and reference light│
└──────────────────────────────────────────────┘
                            │
                            ▼
┌──────────────────────────────────────────────┐
│  Set Gaussian window having different center position│
│ depending on depth of sample through optical detector,│ ── S105
│    and apply set Gaussian window to generate optical│
│          coherence tomographic image based on valid│
│        interference signal for each depth of sample│
└──────────────────────────────────────────────┘
                            │
                            ▼
                        ( End )
```

FIG. 7A

FIG. 7B

FIG. 7C

FIG. 7D

## FIG. 8A

FIG. 8B

FIG. 8C

FIG. 8D

# FIG. 9

# FIG. 10

FIG. 11

FIG. 12

# FIG. 13

## INTERNATIONAL SEARCH REPORT

| International application No. |
| --- |
| **PCT/KR2025/001080** |

### A. CLASSIFICATION OF SUBJECT MATTER

**G01N 21/45**(2006.01)i; **G01N 21/31**(2006.01)i; **G01N 21/47**(2006.01)i; **G01B 9/02091**(2022.01)i; **G01J 9/02**(2006.01)i; **A61B 5/00**(2006.01)i

According to International Patent Classification (IPC) or to both national classification and IPC

### B. FIELDS SEARCHED

Minimum documentation searched (classification system followed by classification symbols)

G01N 21/45(2006.01); A61B 3/10(2006.01); A61B 3/14(2006.01); G01N 21/17(2006.01); G02B 21/36(2006.01); G02B 6/293(2006.01); G02B 7/28(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 광간섭 단층 촬영(Optical Coherence Tomography), 색수차(chromatic aberration), 렌즈(lens), 분산(dispersion), 및 시야 심도(depth of focus)

### C. DOCUMENTS CONSIDERED TO BE RELEVANT

| Category* | Citation of document, with indication, where appropriate, of the relevant passages | Relevant to claim No. |
| --- | --- | --- |
| Y | ROMODINA, Maria N. et al. Depth of focus extension in optical coherence tomography using ultra high chromatic dispersion of zinc selenide. Journal of Biophotonics. 13 May 2022 (online publication date), vol. 15, no. 8, pp. 1-7. Retrieved from (URL: https://onlinelibrary.wiley.com/doi/full/10.1002/jbio.202200051).<br>See pages 2-7 and figures 1-4. | 1-15 |
| Y | JP 2012-506060 A (BURNHAM INSTITUTE FOR MEDICAL RESEARCH) 08 March 2012 (2012-03-08)<br>See paragraphs [0027], [0092] and [0145]-[0159] and figures 1, 28-29 and 42-52. | 1-15 |
| A | JP 2020-096866 A (CARL ZEISS MEDITEC INC. et al.) 25 June 2020 (2020-06-25)<br>See paragraphs [0012]-[0236] and figures 1-19. | 1-15 |
| A | CN 215687754 U (GUANGDONG WEIREN MEDICAL TECHNOLOGY CO., LTD.) 01 February 2022 (2022-02-01)<br>See paragraphs [0032]-[0050] and figures 1-8. | 1-15 |

☑ Further documents are listed in the continuation of Box C.     ☑ See patent family annex.

\*   Special categories of cited documents:
"A"   document defining the general state of the art which is not considered to be of particular relevance
"D"   document cited by the applicant in the international application
"E"   earlier application or patent but published on or after the international filing date
"L"   document which may throw doubts on priority claim(s) or which is cited to establish the publication date of another citation or other special reason (as specified)
"O"   document referring to an oral disclosure, use, exhibition or other means
"P"   document published prior to the international filing date but later than the priority date claimed

"T"   later document published after the international filing date or priority date and not in conflict with the application but cited to understand the principle or theory underlying the invention
"X"   document of particular relevance; the claimed invention cannot be considered novel or cannot be considered to involve an inventive step when the document is taken alone
"Y"   document of particular relevance; the claimed invention cannot be considered to involve an inventive step when the document is combined with one or more other such documents, such combination being obvious to a person skilled in the art
"&"   document member of the same patent family

| Date of the actual completion of the international search | Date of mailing of the international search report |
| --- | --- |
| **23 April 2025** | **23 April 2025** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2025/001080** |

| C. | DOCUMENTS CONSIDERED TO BE RELEVANT | |
| --- | --- | --- |
| Category* | Citation of document, with indication, where appropriate, of the relevant passages | Relevant to claim No. |
| A | US 2019-0377134 A1 (BOSTON MEDICAL CENTER CORPORATION) 12 December 2019 (2019-12-12)<br>See paragraphs [0051]-[0182] and figures 1-11. | 1-15 |

Form PCT/ISA/210 (second sheet) (July 2022)

## INTERNATIONAL SEARCH REPORT
### Information on patent family members

International application No.

**PCT/KR2025/001080**

| Patent document cited in search report | | | Publication date (day/month/year) | Patent family member(s) | | | Publication date (day/month/year) |
|---|---|---|---|---|---|---|---|
| JP | 2012-506060 | A | 08 March 2012 | AU | 2009-305771 | A1 | 22 April 2010 |
| | | | | AU | 2009-305771 | B2 | 15 August 2013 |
| | | | | CA | 2728662 | A1 | 22 April 2010 |
| | | | | CA | 2728662 | C | 16 June 2020 |
| | | | | CN | 102245749 | A | 16 November 2011 |
| | | | | EP | 2344020 | A1 | 20 July 2011 |
| | | | | EP | 2344020 | B1 | 20 May 2020 |
| | | | | EP | 2350244 | A1 | 03 August 2011 |
| | | | | EP | 2353042 | A1 | 10 August 2011 |
| | | | | EP | 3725212 | A1 | 21 October 2020 |
| | | | | JP | 2012-505669 | A | 08 March 2012 |
| | | | | JP | 5778579 | B2 | 16 September 2015 |
| | | | | US | 2010-0094127 | A1 | 15 April 2010 |
| | | | | US | 2010-0172020 | A1 | 08 July 2010 |
| | | | | US | 2012-0021497 | A1 | 26 January 2012 |
| | | | | US | 2014-0018669 | A1 | 16 January 2014 |
| | | | | US | 8478387 | B2 | 02 July 2013 |
| | | | | US | 8760756 | B2 | 24 June 2014 |
| | | | | US | 9462950 | B2 | 11 October 2016 |
| | | | | WO | 2010-044870 | A1 | 22 April 2010 |
| | | | | WO | 2010-045386 | A1 | 22 April 2010 |
| | | | | WO | 2010-045392 | A1 | 22 April 2010 |
| JP | 2020-096866 | A | 25 June 2020 | EP | 3155361 | A2 | 19 April 2017 |
| | | | | EP | 3155361 | B1 | 14 August 2024 |
| | | | | JP | 2017-522066 | A | 10 August 2017 |
| | | | | JP | 6941696 | B2 | 29 September 2021 |
| | | | | US | 10799111 | B2 | 13 October 2020 |
| | | | | US | 11890052 | B2 | 06 February 2024 |
| | | | | US | 2016-0206193 | A1 | 21 July 2016 |
| | | | | US | 2017-0105618 | A1 | 20 April 2017 |
| | | | | US | 2021-0007601 | A1 | 14 January 2021 |
| | | | | US | 2024-0115128 | A1 | 11 April 2024 |
| | | | | WO | 2015-024663 | A1 | 26 February 2015 |
| | | | | WO | 2015-189174 | A2 | 17 December 2015 |
| | | | | WO | 2015-189174 | A3 | 04 February 2016 |
| CN | 215687754 | U | 01 February 2022 | None | | | |
| US | 2019-0377134 | A1 | 12 December 2019 | US | 10732354 | B2 | 04 August 2020 |
| | | | | WO | 2019-236888 | A1 | 12 December 2019 |

Form PCT/ISA/210 (patent family annex) (July 2022)